# FASCICULE DE BREVET EUROPEEN

(11) **EP 0 824 531 B1**
(45) Date de publication et mention de la délivrance du brevet: **22.03.2000**
(21) Numéro de dépôt: 96919883.7
(22) Date de dépôt: 09.05.1996
(51) Int. Cl.: C07D 307/85, C07D 405/04

(54) **PROCEDE DE PREPARATION D'UN DERIVE D'ACIDE BENZOFURANE CARBOXYLIQUE OPTIQUEMENT PUR, ET SON UTILISATION POUR PREPARER L'EFAROXAN**
VERFAHREN ZUR HERSTELLUNG EINES OPTISCH REINEN BENZOFURANCARBONSÄUREDERIVATES UND DESSEN VERWENDUNG ZUR HERSTELLUNG VON EFAROXAN
METHOD FOR PREPARING AN OPTICALLY PURE BENZOFURAN CARBOXYLIC ACID AND USE THEREOF FOR PREPARING EFAROXAN

(30) Priorité: 10.05.1995 FR 9505513
(43) Date de publication de la demande: 25.02.1998
(73) Titulaire: PIERRE FABRE MEDICAMENT, 92100 Boulogne (FR)
(72) Inventeur: IMBERT, Thierry, F-81290 Viviers-les-Montagnes (FR); MAYER, Patrick, F-81100 Castres (FR)
(74) Mandataire: Ahner, Francis
(86) Numéro de dépôt international: FR9600697
(87) Numéro de publication internationale: WO9635682

(56) Documents cités:
- EP-A- 0 310 745
- WO-A-92/05171
- GB-A- 2 102 422
- JOURNAL OF HETEROCYCLIC CHEMISTRY, vol. 24, no. 2, Mars 1987, PROVO US, pages 495-496, XP002010279 C. R. EDWARDS ET AL.: "A Practical Synthesis of 2,3-Dihydro-2-benzofurancarboxylic Acid: A General Route to 2,3-Dihydrobenzofurans "

## Description

La présente invention concerne un procédé de préparation d'un dérivé d'acide 2-éthyl-2,3-dihydro-benzofurane-carboxylique optiquement pur, le dérivé obtenu et son utilisation pour la préparation du dérivé du 2-[2-(2-éthyl-2,3-dihydro-benzofuranyl)]-2-imidazoline optiquement pur correspondant, en particulier de l'efaroxan.

Les dérivés du 2-[2-(2-éthyl-2,3-dihydro-benzofuranyl)]-2-imidazoline, en particulier l'efaroxan, sont des dérivés antagonistes des récepteurs a2-adrénergiques, décrits dans la demande de brevet européen N° 0 071 368 (Reckitt & Colman) pour le traitement de la dépression ou de la migraine. Ils sont également décrits pour le traitement de la maladie de Parkinson et des troubles neurodégénératifs comme la maladie d'Alzheimer dans les demandes de brevet WO N° 95/00145 et N° 95/01791 (Pierre Fabre Médicament).

Leurs énantiomères sont pour leur part décrits dans la demande de brevet WO N° 92/05171, le (-) efaroxan étant en particulier décrit pour traiter le diabète, comme agent bloqueur des canaux potassiques. Ils sont obtenus par résolution du racémique avec l'acide dibenzoyl-tartrique, au stade final de la synthèse.

La présente invention concerne un nouveau procédé de synthèse stéréospécifique des dérivés optiquement purs du 2-[2-(2-éthyl-2,3-dihydrobenzofuranyl)]-2-imidazoline, de formule générale I 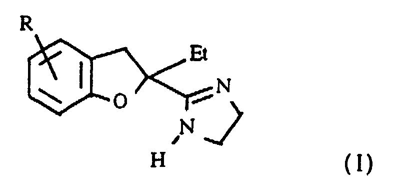 dans laquelle R représente un atome d'hydrogène, un halogène, un radical alkyle inférieur, alkoxy inférieur ou hydroxy, à partir d'un dérivé d'acide 2-éthyl-2,3-dihydro-benzofurane-carboxylique optiquement pur correspondant, et les dérivés optiquement purs obtenus par ce procédé.

La synthèse des dérivés de formule générale I est plus particulièrement décrite dans la demande de brevet EP-A-0 071 368, et consiste à transformer le dérivé d'acide 2-éthyl-2,3-dihydro-benzofurane-carboxylique correspondant en amide de formule générale III, 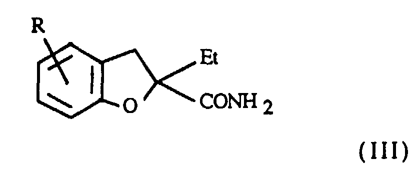 dans laquelle R est défini ci-dessus,
puis en dérivé cyano correspondant de formule générale IV, par déshydratation, 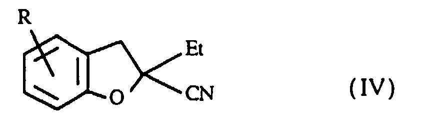 lequel est ensuite transformé en dérivé de formule générale I, selon les techniques usuelles avec de l'éthylène diamine.

Le procédé de synthèse spécifique selon l'invention reprend ce schéma général avec, comme acide de départ, le dérivé d'acide 2-éthyl-2,3-dihydro-benzofurane-carboxylique optiquement pur correspondant.

La présente invention concerne donc tout d'abord le procédé de préparation de l'acide de départ de formule générale Il 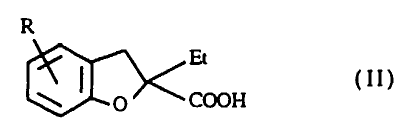 dans laquelle R représente un atome d'hydrogène, un halogène, un radical alkyle inférieur, alkoxy inférieur ou hydroxy,
procédé pour lequel on effectue une résolution du mélange racémique par cristallisation sélective avec l'énantiomère optiquement pur approprié du 2-phénylglycinol, dans un solvant approprié, puis on isole et on récupère l'acide de formule II optiquement pur cristallisé.

Par énantiomère optiquement pur approprié du 2-phénylglycinol, on entend l'énantiomère de l'acide permettant une bonne séparation des sels diastéréoisomères obtenus, l'un étant plus stable que l'autre, et cristallisant. Ainsi, on obtient l'acide de formule générale II de configuration R, par cristallisation sélective avec le S(+)-2-phénylglycinol et inversement l'acide de configuration S avec le R(-)-2-phénylglycinol.

Par solvant approprié, on entend tout solvant susceptible de favoriser la cristallisation sélective d'un sel énantiomère, tout en conservant l'autre en solution. Il sera avantageusement choisi parmi l'acétone, l'acétate d'éthyle, la méthyl-éthyl-cétone et leurs mélanges.

La préparation de l'acide de formule générale II racémique est décrite dans le *Journal of Heterocyclic Chemistry* (1987, 24, 495).

Le principe de la cristallisation sélective par formation de sels diastéréoisomères est un principe connu. Toutefois, sa réalisation reste une opération difficile et hasardeuse, en particulier lorsqu'il s'agit d'obtenir une pureté optique élevée, c'est-à-dire supérieure ou égale à 95 %.

Une méthode de séparation d'énantiomères d'acides tels que l'acide de formule générale II a été décrite, non pas par cristallisation sélective de sels diastéréoisomères, mais par estérification avec le menthol optiquement actif (*Chem. Pharm. Bull.,* 1988, 36, 902), ce qui nécessite une étape supplémentaire d'hydrolyse de l'ester diastéréoisomère isolé.

Différentes amines chirales peuvent être employées pour former un sel diastéréoisomère avec l'acide de formule générale II, comme par exemple l'α-méthyl-benzyl-amine, ou des amino-alcools tels le phénylalaninol ou le prolinol optiquement actifs.

Toutefois, des essais de résolution de l'acide 2-éthyl-2,3-dihydrobenzofurane-carboxylique avec les énantiomères de ces trois alcools se sont révélés infructueux, seul le 2-phénylglycinol optiquement pur permettant une séparation nette des sels diastéréoisomères par cristallisation sélective.

Le S(+)-2-phénylglycinol est un amino-alcool facilement accessible par réduction de la S(+)-phénylglycine (*Tetrahedron Lett.,* 1992, 33, 5517), et son utilisation pour la séparation de certains acides racémiques de structure éloignée des acides de formule générale Il est notamment décrite dans les demandes de brevets japonais N° 58 029 719, N° 53 018 529 et N° 03 095 138.

Les résultats de ces essais de cristallisation dans l'acétate d'éthyle ou l'acétone sont reportés sur le Tableau I ci-après, la pureté des sels cristallisés étant vérifiée par HPLC (colonne : chirale x (250 x 4 mm) β-cyclodextrine; éluant : tampon TEAA 1%, pH 4,1, avec 15 % de MeOH).

**TABLEAU I**

| **ALCOOL CHIRAL** | **RESULTAT** | **HPLC** |
|---|---|---|
| R(+)-α-méthylbenzylamine | cristallisation 2 sels | 2 pics d'égale intensité |
| S(-)-phénylalaninol | cristallisation 2 sels | 2 pics d'égale intensité |
| S(+)-prolinol | pas de cristallisation | - |
| R(-)-2-phénylglycinol | cristallisation avec l'énantiomère (-) | 1 seul pic |
| S(+)-2-phénylglycinol | cristallisation avec l'énantiomère (+) | 1 seul pic |

La présente invention concerne également les dérivés de formule générale II, optiquement purs pouvant être obtenus par le procédé ci-dessus, en particulier les dérivés ayant une pureté énantiomérique supérieure ou égale à 95 %, plus particulièrement supérieure à 96,5 %.

A partir de cet acide optiquement pur, on prépare les dérivés de formule générale I correspondants, en conservant la configuration absolue du produit.

Le schéma réactionnel de préparation des dérivés de formule générale I, à partir de l'acide racémique, est donné en page suivante pour la préparation du R(+)-efaroxan, c'est-à-dire R représente un atome d'hydrogène.

Le mélange racémique de l'acide formule II où R = H, est placé dans l'acétone, l'acétate d'éthyle, ou la méthyléthylcétone en présence d'une quantité stoechiométrique de S(+)-phénylglycinol. Après cristallisation, on isole le sel diastéréoisomère de l'acide (+) 2-éthyl-2,3-dihydro-benzofurane-carboxylique (A+) et du S(+)-phénylglycinol (B+). L'autre sel diastéréoisomère formé entre l'acide (-)-2-éthyl-2,3-dihydro-benzofurane-carboxylique et du S(+)-phénylglycinol reste en solution. Le spectre de RMN de l'entité (A+) (B+) se présente comme composé unique.

Par passage à la forme acide libre en présence d'acide chlorhydrique, on obtient l'acide (+)-2-éthyl-2,3-dihydro-benzofurane-carboxylique énantiomériquement pur. Cet acide est transformé en ester par traitement par SOCl₂/MeOH, puis l'ester méthylique obtenu est traité par l'ammoniaque pour former l'amide qui, par déshydratation avec P₂O₅ dans le toluène, fournit le nitrile correspondant. Le dernier stade de la synthèse consiste en la préparation de l'imidate à partir du nitrile, par réaction avec une quantité catalytique de méthylate de sodium, lequel est ensuite traité par de l'éthylènediamine dans une solution d'isopropanol et d'acide chlorhydrique, pour obtenir l'imidazoline recherchée.

Le rendement global de la préparation de l'isomère dextrogyre de l'acide-2-éthyl-2,3-dihydro-benzofurane-2-carboxylique est de 50 % à partir de son racémique, et la préparation du (+)-2-[2-(2-éthyl-2,3-dihydrobenzofuranyl] -2-imidazoline sous forme de chlorhydrate est de 50 %.

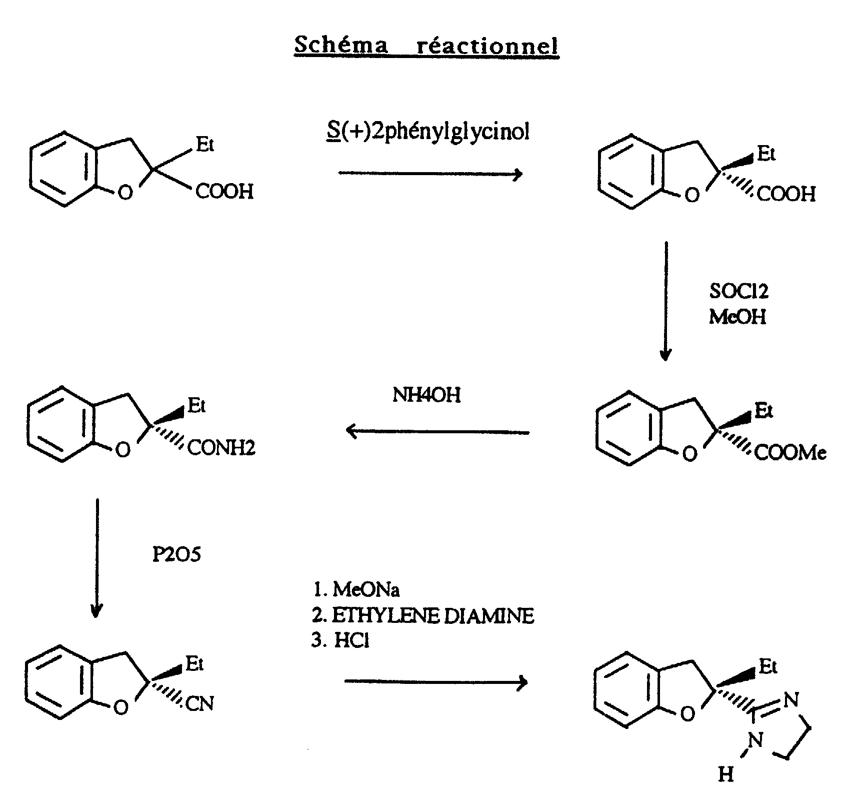

D'autres caractéristiques du procédé selon l'invention apparaîtront à la lumière des exemples ci-après de préparation sélective du R(+)-efaroxan.

### Exemple 1: Acide (+)-2-éthyl-2,3-dihydro-2-benzofurane-carboxylique

L'acide racémique 2-éthyl-2,3-dihydro-2-benzofurane-carboxylique (6 g ; 31,2 mmoles) est dissous dans 50 ml d'acétate d'éthyle et mélangé avec une solution de S(+)-2phénylglycinol (4,29 g ; 31,2 mmoles) dans 100 ml d'acétate d'éthyle. Les cristaux sont filtrés, recristallisés 2 fois dans la méthyléthylcétone pour conduire à une pureté énantiomérique de 96,6 % ; [α]D = + 55,9° (C = 0,3; MeOH). L'acide libre est obtenu par extraction dans CH₂Cl₂ et HCl N pour fournir 1,52 g (50 %) de cristaux qui sont engagés dans l'étape de l'exemple 2.

En partant du même acide racémique mais en utilisant le R(-)-2-phénylglycinol, on obtient le sel diastéréoisomère après deux recristallisations dans l'acétone et la méthyléthylcétone. On obtient des cristaux dont le pouvoir rotatoire est : [α]D = - 55,70° (C = 0,26 ; MeOH).

### Exemple 2 : (+)-2-éthyl-2,3-dihydro-2-benzofurane-carboxylate de méthyle

L'acide énantiomériquement pur obtenu à l'exemple 1 est traité par 0,5 ml de SOCl₂ dans 100 ml de méthanol, à température ordinaire pendant une nuit, par évaporation, on obtient 1,5 g d'ester méthylique.

### Exemple 3 : (+)-2-éthyl-2,3-dihydro-2-benzofurane-carboxamide

L'ester du stade précédent est placé sous agitation avec une solution de 50 ml d'ammoniaque concentrée, à température ordinaire. Après évaporation, on obtient 1,38 g de composé amide brut, qui est engagé dans l'étape suivante.

### Exemple 4 : (+)-2-éthyl-2,3-dihydro-2-cyano-benzofurane

L'amide de l'exemple 3 est traité par 5 g de P₂O₅ dans 50 ml de toluène au reflux pendant 16 h. Après décantation, extraction usuelle, on obtient 1,06 g de nitrile, qui est engagé directement dans l'étape suivante.

### Exemple 5 : (+)-2-[2-(2-éthyl-2,3-dihydro-benzofuranyl)]-2-imidazoline, chlorhydrate

Le nitrile obtenu à l'exemple 4 (1 g) est placé dans l'éthanol (50 ml) et traité par une quantité catalytique de méthylate de sodium a 0°C, puis laissé pendant 6 jours à température ordinaire. A la fin de la réaction (CCM), 0,582 ml d'éthylènediamine sont ajoutés dans le milieu réactionnel suivi d'une solution 5N d'isopronanol saturée de gaz chlorhydrique. L'agitation est maintenue 4 jours puis le mélange réactionnel est extrait par CH₂Cl₂ et une solution de soude 1N, on obtient alors 702 mg du composé sous forme de base, dont on forme le chlorhydrate dans l'éther par addition de 0,62 ml d'isopropanol, HCl (5N). Une recristallisation dans l'acétonitrile fournit le chorhydrate sous forme de cristaux purs.
F = 246°C ; [a]D = + 99,32 (C = 0,29 ; MeOH)
IR (KBr) : ν cm⁻¹ = 2977, 2901, 1603, 1480, 1460
¹H-RMN(DMSO): δ = 0,90 (t, 3H, J = 7,2 Hz, CH₃) ; 1,95 - 2,2 (m, 2H, CH₂ éthyl) ; 3,35 (d, 1H, J = 16,7 Hz, ArCH_{A}) ; 3,57 (d = 1H, J = 16,7 Hz, ArCH_{B}) ; 3,85 (s, 4H, 2CH₂ imidazoline) ; 6,86 - 6,95 (m, 2H, H₅ - H₇) ; 7,13 - 7,25 (m, 2H, H₄ - H₆) ; 9,20 (d, 1H, NH).

Pureté énantiomérique : 99,9 %.

Des essais de séparations des acides racémiques II ayant un substituant R = 4 - F et R = 5 - F conduisent à l'obtention des acides II correspondants, dextrogyres, optiquement purs. Ces acides sont utilisés à la préparation des composés dérivés de formule générale I correspondants énantiomériquement purs, également partie de la présente invention :
(+)-2-[2-(2-éthyl-4-fluoro-2,3-dihydro-benzofuranyl)]-2-imidazoline, chlorhydrate ;
(+)-2-[2-(2-éthyl-5-fluoro-2,3-dihydro-benzofuranyl)]-2-imidazoline, chlorhydrate.

## Revendications

1. Procédé de préparation d'un dérivé d'acide 2-éthyl-2,3-dihydro-benzofurane-carboxylique optiquement pur, de formule générale II 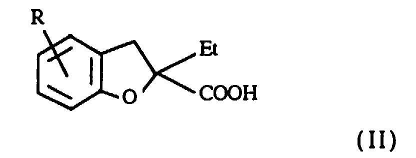 dans laquelle R représente un atome d'hydrogène, un halogène, un radical alkyle inférieur, alkoxy inférieur ou hydroxy,
procédé pour lequel on effectue une résolution du mélange racémique par cristallisation sélective avec l'énantiomère optiquement pur approprié du 2-phénylglycinol, dans un solvant approprié, puis on isole et on récupère l'acide de formule II optiquement pur cristallisé.

2. Procédé selon la revendication 1, caractérisé en ce que l'on obtient l'acide de formule générale II de configuration R(+), par cristallisation sélective avec le S(+)-2-phénylglycinol.

3. Procédé selon l'une des revendications 1 ou 2, caractérisé en ce que le solvant est séléctionné parmi l'acétone, l'acétate d'éthyle, la méthyl-éthyl-cétone et leurs mélanges.

4. Acide 2-éthyl-2,3-dihydro-benzofurane-carboxylique optiquement pur, de formule générale II pouvant être obtenu par le procédé selon l'une des revendications 1 à 3.

5. Acide 2-éthyl-2,3-dihydro-benzofurane-carboxylique optiquement pur, de formule générale II optiquement pur, 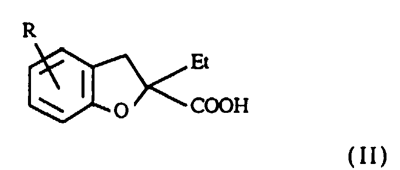 dans laquelle R représente un atome d'hydrogène, un halogène, un radical alkyle inférieur, alkoxy inférieur ou hydroxy,
caractérisé en ce qu'il a une pureté énantiomérique supérieure ou égale à 95 %, en particulier supérieure à 96,5 %.

6. Acide R(+)-2-éthyl-2,3-dihydro-benzofurane-carboxylique optiquement pur selon l'une des revendications 4 ou 5.

7. Procédé de préparation d'un dérivé optiquement pur du 2-[2-(2-éthyl-2,3-dihydro-benzofuranyl)]-2-imidazoline de formule générale I 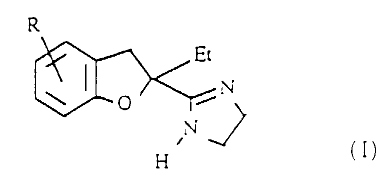 dans laquelle R représente un atome d'hydrogène, un halogène, un radical alkyle inférieur, alkoxy inférieur ou hydroxy,
par transformation du dérivé d'acide 2-éthyl-2,3-dihydro-benzofurane-carboxylique optiquement pur, de formule générale Il correspondant, défini selon l'une des revendications 4 à 6.

8. Procédé selon la revendication 7, caractérisé en ce que le dérivé du 2-[2-(2-éthyl-2,3-dihydro-benzofuranyl)]-2-imidazoline de formule générale I et l'acide 2-éthyl-2,3-dihydro-benzofurane-carboxylique optiquement pur de formule générale II, correspondant, sont chacun de configuration R.

9. Procédé selon la revendication 8, caractérisé en ce que R représente un atome d'hydrogène, et le dérivé du 2-[2-(2-éthyl-2,3-dihydrobenzofuranyl)]-2-imidazoline de formule générale I et l'acide 2-éthyl-2,3-dihydro-benzofurane-carboxylique optiquement pur de formule générale II, correspondant, sont chacun de configuration R et dextrogyres (+).

## Patentansprüche

1. Verfahren zur Herstellung eines optisch reinen Derivats von 2-Ethyl-2,3-dihydrobenzofurancarbonsäure der allgemeinen Formel II 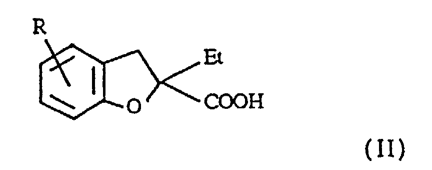 wobei R ein Wasserstoffatom, ein Halogen, einen niedrigen Alkyl-, niedrigen Alkoxy- oder Hydroxyrest darstellt,
Verfahren, bei dem man eine Trennung der racemischen Mischung durch selektive Kristallisation mit dem geeigneten optisch reinen Enantiomeren von 2-Phenylglycinol in einem geeigneten Lösungsmittel durchführt, und anschließend die optisch reine kristallisierte Form der Säure der Formel II abtrennt und isoliert.

2. Verfahren nach Anspruch 1, dadurch charakterisiert, daß man die Säure der allgemeinen Formel II der Konfiguration R(+) durch selektive Kristallisation mit S(+)-2-Phenylglycinol erhält.

3. Verfahren nach einem der Ansprüche 1 oder 2, dadurch charakterisiert, daß das Lösungsmittel ausgewählt wird aus Aceton, Ethylacetat, Methylethylketon und ihren Gemischen.

4. Optisch reine 2-Ethyl-2,3-dihydrobenzofürancarbonsäure der allgemeinen Formel II, erhältlich durch das Verfahren nach einem der Ansprüche 1 bis 3.

5. Optisch reine 2-Ethyl-2,3-dihydrobenzofurancarbonsäure der allgemeinen Formel II in optisch reiner Form, 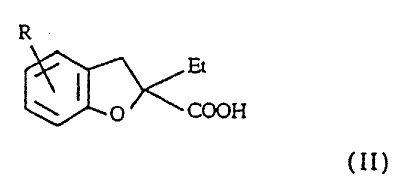 wobei R ein Wasserstoffatom, ein Halogen, einen niedrigen Alkyl-, niedrigen Alkoxy- oder Hydroxyrest darstellt,
dadurch charakterisiert, daß sie eine Enantiomerenreinheit von mehr oder gleich 95 %, insbesondere mehr als 96,5 % aufweist.

6. Optisch reine R(+)-2-Ethyl-2,3-dihydro-benzofurancarbonsäure nach einem der Ansprüche 4 oder 5.

7. Verfahren zur Herstellung eines optisch reinen Derivats von 2-[2-(2-Ethyl-2,3-dihydro-benzofuranyl)]-2-imidazolin der allgemeinen Formel I 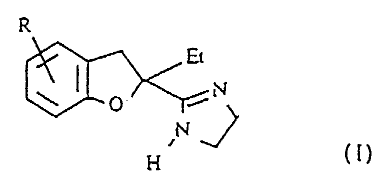 wobei R ein Wasserstoffatom, ein Halogen, einen niedrigen Alkyl-, niedrigen Alkoxy- oder Hydroxyrest darstellt,
durch Umwandlung eines Derivats der entsprechenden optisch reinen 2-Ethyl-2,3-dihydrobenzofurancarbonsäure der allgemeinen Formel II, die nach einem der Ansprüche 4 bis 6 definiert ist.

8. Verfahren nach Anspruch 7, dadurch charakterisiert, daß das Derivat von 2-[2-(2-Ethyl-2,3-dihydro-benzofuranyl)]-2-imidazolin der allgemeinen Formel I und die entsprechende optisch reine 2-Ethyl-2,3-dihydro-benzofurancarbonsäure der allgemeinen Formel II jeweils die Konfiguration R aufweist.

9. Verfahren nach Anspruch 8, dadurch charakterisiert, daß R ein Wasserstoffatom darstellt, und das Derivat von 2-[2-(2-Ethyl-2,3-dihydrobenzofüranyl)]-2-imidazolin der allgemeinen Formel I und die entsprechende optisch reine 2-Ethyl-2,3-dihydrobenzofurancarbonsäure der allgemeinen Formel II jeweils die Konfiguration R aufweisen und rechtsdrehend (+) sind.

## Claims

1. Process for the preparation of an optically pure 2-ethyl-2,3-dihydrobenzofurancarboxylic acid derivative of general formula II 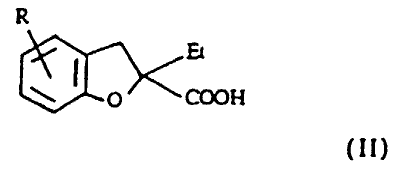 in which R represents a hydrogen or halogen atom or a lower alkyl, lower alkoxy or hydroxyl radical,
in which process the racemic mixture is resolved by selective crystallization with the appropriate optically pure enantiomer of 2-phenylglycinol, in a suitable solvent, after which the crystallized optically pure acid of formula II is recovered.

2. Process according to Claim 1, characterized in that the acid of general formula II of R(+) configuration is obtained by selective crystallization with S (+)-2-phenylglycinol.

3. Process according to either of Claims 1 and 2, characterized in that the solvent is selected from acetone, ethyl acetate, methyl ethyl ketone and mixtures thereof.

4. Optically pure 2-ethyl-2,3-dihydrobenzofurancarboxylic acid of general formula II which can be obtained by the process according to one of Claims 1 to 3.

5. Optically pure 2-ethyl-2,3-dihydrobenzofurancarboxylic acid, which is optically pure, of general formula II, 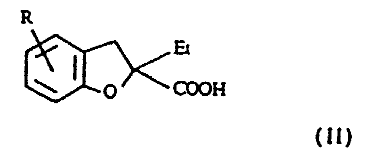 in which R represents a hydrogen or halogen atom or a lower alkyl, lower alkoxy or hydroxyl radical,
characterized in that it has an enantiomeric purity greater than or equal to 95%, in particular greater than 96.5%.

6. Optically pure R (+)-2-ethyl-2,3-dihydrobenzofurancarboxylic acid according to either of Claims 4 and 5.

7. Process for the preparation of an optically pure 2-[2-(2-ethyl-2,3-dihydrobenzofuryl)]-2-imidazoline derivative of general formula I 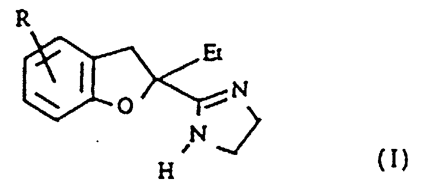 in which R represents a hydrogen or halogen atom or a lower alkyl, lower alkoxy or hydroxyl radical,
by conversion of the optically pure 2-ethyl-2,3-dihydrobenzofurancarboxylic acid derivative of corresponding general formula II, defined according to one of Claims 4 to 6.

8. Process according to Claim 7, characterized in that the 2-[2-(2-ethyl-2,3-dihydrobenzofuryl)]-2-imidazoline derivative of general formula I and the corresponding optically pure 2-ethyl-2,3-dihydrobenzofurancarboxylic acid of general formula II are each of R configuration.

9. Process according to Claim 8, characterized in that R represents a hydrogen atom and the 2-[2-(2-ethyl-2,3-dihydrobenzofuryl)]-2-imidazoline derivative of general formula I and the corresponding optically pure 2-ethyl-2,3-dihydrobenzofurancarboxylic acid of general formula II are each of R configuration and dextrorotatory (+).
